# DEMANDE DE BREVET EUROPEEN

(11) **EP 4 799 653 A1**
(43) Date de publication de la demande: **02.09.2026**
(21) Numéro de dépôt: 26161371.5
(22) Date de dépôt: 27.02.2026
(51) Int. Cl.: A61L 2/10

(54) **ENCEINTE DE DÉSINFECTION D'UN INSTRUMENT MÉDICAL**

(30) Priorité: 28.02.2025 FR 2502034
(71) Demandeur: Germitec, 33000 Bordeaux (FR)
(72) Inventeur: POULON, Fanny, 91440 BURES SUR YVETTE (FR); HAMDOU, Aimrane, 33800 BORDEAUX (FR)
(74) Mandataire: Lavoix

(57) **Abrégé**

La présente invention concerne un enceinte (10) de désinfection d'un instrument médical (12), l'enceinte (10) comprenant :
- un premier caisson (16) définissant un premier logement (20) fermé par une première partition (22), et
- un deuxième caisson (18) définissant un deuxième logement (32) fermé par une deuxième partition (34).

Le deuxième caisson (18) est mobile par rapport au premier caisson (16) entre une position ouverte et une position fermée.

Dans la position fermée, la première partition (22) et la deuxième partition (34) définissent entre elles un volume de désinfection fermé.

L'enceinte (10) comprenant en outre un dispositif de génération de rayonnements UV configuré pour générer des rayonnements UV dans le volume de désinfection, le dispositif de génération de rayonnements UV étant arrangé dans au moins l'un du premier logement (20) et du deuxième logement.

## Description

La présente invention concerne une enceinte de désinfection d'un instrument médical.

Afin d'éviter toutes contaminations, il convient de désinfecter un instrument médical entre deux utilisations.

A cet effet, il est connu de désinfecter des instruments médicaux dans des enceintes de désinfection définissant un volume de désinfection fermé. De telles enceintes comprennent par exemple un dispositif de génération de rayonnements UV (Ultra-violet) permettant notamment d'effectuer une désinfection de ces instruments médicaux, et plus particulièrement une désinfection haut-niveau.

Certains instruments médicaux, tels que des sondes médicales par exemple, comprennent typiquement au moins une partie active et généralement au moins une partie de raccordement sous forme de câble.

Il est connu de suspendre ces instruments médicaux dans l'enceinte de désinfection, par exemple à l'aide d'une potence de suspension comprenant par exemple une molette ou une bague adaptée pour maintenir une portion de la partie de raccordement.

Cependant, de telles enceintes ne sont pas adaptées aux instruments médicaux sans câble de raccordement, tels que les sondes portables par exemple.

Par ailleurs, de telles enceintes sont peu adaptées à la désinfection de sondes de grande taille et/ou asymétriques.

En effet, pour de telles sondes, il existe un risque que la sonde touche l'une des parois de l'enceinte, créant ainsi une zone non-désinfectées, communément appelée « cold spot » (se traduisant par « point froid »).

Par ailleurs, un tel contact entre la sonde et les parois peut entrainer un risque d'endommagement des sources UV arrangées sur les parois.

Un des buts de l'invention est alors de pallier ces inconvénients, et en particulier de proposer une enceinte de désinfection résistante et apte à assurer une désinfection haut-niveau d'un instrument médical, tout en étant compatible avec une grande variété d'instruments médicaux.

A cet effet, l'invention a pour objet une enceinte de désinfection d'un instrument médical, l'enceinte comprenant :
- un premier caisson définissant un premier logement fermé par une première partition, et
- un deuxième caisson définissant un deuxième logement fermé par une deuxième partition;

le deuxième caisson étant mobile par rapport au premier caisson entre une position ouverte et une position fermée,
dans la position fermée, la première partition et la deuxième partition définissant entre elles un volume de désinfection fermé ;
l'enceinte comprenant en outre un dispositif de génération de rayonnements UV configuré pour générer des rayonnements UV dans le volume de désinfection, le dispositif de génération de rayonnements UV étant arrangé dans au moins l'un du premier logement et du deuxième logement.

En effet, grâce aux partitions définissant un support pour l'instrument médical, une telle enceinte permet de désinfecter une large gamme d'instruments, de tailles et de formes variées, qu'ils soient équipés ou non de câbles.

En outre, les partitions évitent que l'instrument médical touche les parois de l'enceinte, ce qui permet de limiter la création de zones non désinfectées et d'assurer ainsi une désinfection satisfaisante.

Par ailleurs, les partitions permettent de protéger le dispositif de génération de rayonnements UV, et d'ainsi éviter son endommagement.

Suivant d'autres aspects avantageux de l'invention, l'enceinte de désinfection comprend une ou plusieurs des caractéristiques suivantes, prises isolément ou suivant toutes les combinaisons techniquement possibles :
- L'au moins l'une de la première partition et de la deuxième partition comprend une partie centrale apte à définir un logement de réception de l'instrument médical.
- L'une de la première et de la deuxième partition est transparente aux rayonnements UV.
- Au moins l'une de la première et de la deuxième partition a une transmittance aux rayonnements UV supérieure à 30%, et de préférence supérieure à 50%.
- Au moins l'une de la première et de la deuxième partition est fabriquée en quartz.
- L'enceinte comprend en outre une charnière agencée entre le premier caisson et le deuxième caisson et autorisant la mobilité du deuxième caisson par rapport au premier caisson.
- Le volume de désinfection est cylindrique.
- Le dispositif de génération de rayonnements UV comprend une pluralité de sources UV arrangées dans au moins un du premier logement et du deuxième logement.
- Le premier caisson comprend au moins un composant électronique additionnel agencé dans le premier logement.
- L'au moins un composant électronique est une unité d'identification ou une unité de contrôle de la désinfection.

L'invention apparaîtra plus clairement à la lecture de la description qui va suivre, donnée uniquement à titre d'exemple non limitatif, et faite en référence aux dessins dans lesquels :
La Figure 1 est une représentation schématique en perspective d'une enceinte de désinfection selon l'invention, l'enceinte étant en position ouverte, et
La Figure 2 est une représentation schématique en coupe de l'enceinte de désinfection de la Figure 1 en position fermée.

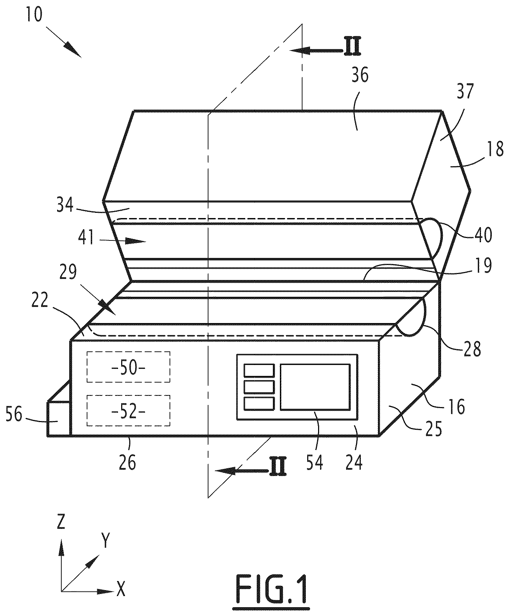

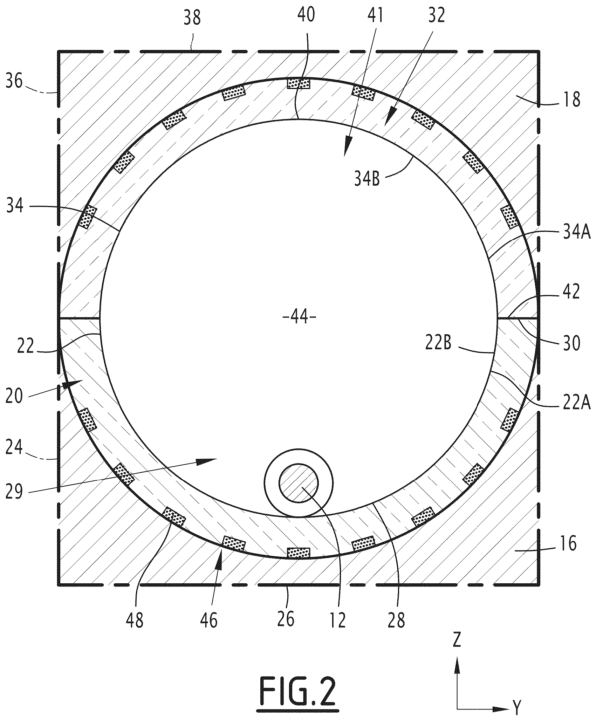

Les Figures 1 et 2 illustrent un exemple d'une enceinte de désinfection 10 selon l'invention.

L'enceinte de désinfection 10 est par exemple configurée pour désinfecter en entier un instrument médical 12.

Par désinfection en entier de l'instrument médical 12, on entend une désinfection de toutes les parties de l'instrument médical 12 dans l'enceinte de désinfection 10.

L'instrument médical 12 est par exemple une sonde médicale, notamment une sonde médicale échographique, par exemple une sonde endocavitaire ou une sonde transoephagienne. La sonde médicale est par exemple une sonde linéaire ou une sonde convexe. La sonde médicale est par exemple une sonde pédiatrique ou pour adulte.

L'instrument médical 12 comprend par exemple une partie active. La partie active est par exemple destinée à être en contact direct ou indirect avec le patient lors de son utilisation, en fonction de l'application médicale.

La partie active de l'instrument médical 12 a par exemple une longueur comprise entre 10 cm et 40 cm.

L'instrument médical 12 est par exemple un instrument médical sans fil.

Par « sans fil », on entend que l'instrument médical ne comprend pas de partie de raccordement sous forme de câble par exemple.

En particulier, l'instrument médical 12 comprend par exemple une batterie et/ou un module de communication sans fil.

Alternativement, l'instrument médical 12 comprend, en outre, une partie de raccordement et par exemple également un connecteur. La partie de raccordement est par exemple un câble de raccordement reliant la partie active au connecteur. Le connecteur est par exemple configuré pour être connecté à une unité de mesure et/ou d'affichage, de sorte notamment à collecter et/ou afficher des informations captées par la partie active.

L'enceinte 10 comprend un premier caisson 16 et un deuxième caisson 18.

Le deuxième caisson 18 est mobile par rapport au premier caisson 16 entre une position ouverte, illustrée sur la Figure 1, et une position fermée, illustrée sur la Figure 2
Dans l'exemple particulier illustré sur les Figures, l'enceinte 10 est de forme parallélépipède rectangle en position fermée, en particulier définissant un repère orthonormé associé comprenant un axe longitudinal X, un axe transversal Y et un axe vertical Z.

Par exemple, l'enceinte 10 comprend notamment une charnière 19 agencée entre le premier caisson 16 et le deuxième caisson 18 et autorisant la mobilité du deuxième caisson 18 par rapport au premier caisson 16.

En particulier, le deuxième caisson 18 est par exemple pivotable selon un axe de pivot parallèle à l'axe longitudinal X par rapport au premier caisson 16 entre la position ouverte et la position fermée.

Le premier caisson 16 définit un premier logement 20 fermé par une première partition 22.

De préférence, le premier caisson 16 comprend au moins une paroi 24, 25, 26.

Par exemple, le premier caisson 16 comprend une pluralité de parois 24, 25, 26 définissant entre elles le premier logement 20.

Dans le mode de réalisation de l'invention illustré sur les Figures 1 et 2, la pluralité de parois du premier caisson 16 comprend par exemple deux parois longitudinales 24 s'étendant perpendiculairement à l'axe transversal Y, deux parois transversales 25 s'étendant perpendiculairement à l'axe longitudinal X et reliant chacune les deux premières parois longitudinales 24 selon l'axe transversal Y, et un fond 26 s'étendant perpendiculairement à l'axe vertical Z et reliant les deux parois longitudinales 24 selon l'axe transversal Y et les deux parois transversales 25 selon l'axe longitudinal X.

Chacune des parois 24, 25, 26 est par exemple une plaque, notamment sensiblement rectangulaire.

Dans une variante non-illustrée, le premier caisson 16 comprend une unique paroi délimitant le premier logement 20, qui est par exemple demi-cylindrique ou demi-sphérique.

La première partition 22 ferme le premier logement 20.

Autrement dit, le premier logement 20 est un volume fermé.

Plus précisément, le premier logement 20 est délimité entre la ou les parois 24, 25, 26 du premier caisson 16 et la première partition 22.

Dans le mode de réalisation particulier illustré, la première partition 22 s'étend notamment en regard du fond 26.

De préférence, la première partition 22 est fixée à chacune des deux parois longitudinales 24 et des deux parois transversales 25, et notamment sur le bord supérieur de chacune de ces quatre parois 24, 25 comme cela est visible sur les Figures.

Par bord supérieur, on entend ici le bord de chacune de ces quatre parois 24, 25 opposé au fond 26.

Comme cela est visible sur les Figures, la première partition 22 comprend une face interne 22A, en particulier orientée vers le premier logement 20, et une face externe 22B opposées à la face interne 22A.

La première partition 22 est de préférence une plaque.

En particulier, l'épaisseur de la première partition 22, c'est-à-dire la distance selon l'axe verticale Z entre les deux faces 22A, 22B, est constante, et par exemple comprise entre 3 mm et 10 mm.

Avantageusement, la première partition 22 comprend une partie centrale 28 apte à définir un logement de réception 29 de l'instrument médical 12.

Par exemple, la partie centrale 28 a une section transversale, c'est-à-dire dans le plan YZ, en forme de demi-cercle, par exemple de diamètre compris entre 10 cm et 30 cm.

Par exemple, le logement de réception 29 est délimité par la face externe 22B de la partie centrale 28 de la première partition 22.

En particulier, le logement de réception 29 est par exemple en forme de demi-cylindre s'étendant selon l'axe longitudinal X.

Alternativement, dans des variantes-illustrées, la partie centrale 28 a une section transversale en forme de U.

Dans un mode de réalisation particulier, la première partition 22 comprend également deux parties latérales 30 reliées entre elles par la partie centrale 28, notamment selon l'axe transversal Y.

En particulier, chacune des parties latérales 30 de la première partition 22 s'étend entre le bord supérieur de l'une des parois longitudinales 24 et la partie centrale 28.

De préférence, chacune des parties latérales 30 s'étend parallèlement au fond 26.

La première partition 22 est de préférence transparente aux rayonnements UV, et notamment aux rayonnements UV-C.

En particulier, la première partition 22 a une transmittance aux rayonnements UV supérieure à 30%, et de préférence supérieure à 50%, et de manière encore plus préférentielle supérieure à 90%.

Par « transmittance », on entend le rapport des rayonnements transmis par la première partition 22 sur les rayonnements incidents sur la première partition 22.

Autrement dit, au moins 30% des rayonnements UV incidents sur la première partition 22, et en particulier sa face interne 22A, sont transmis à travers la première partition 22, et en particulier ressorte par la face externe 22B.

La première partition 22 est préférablement fabriquée en quartz, de préférence du quartz synthétique, tel que par exemple un verre de quartz optique de catégorie JGS1.

Alternativement, la première partition 22 est fabriquée en un polymère, préférablement un copolymère d'oléfine cyclique (COC de l'anglais « Cyclic olefin copolymer »), en particulier du TOPAS^{®} 8007X10, ou un polyméthylpentène, tel que le TPX^{™} RT18, ou de l'éthylène-propylène fluoré.

Le deuxième caisson 18 définit un deuxième logement 32 fermé par une deuxième partition 34.

De préférence, le deuxième caisson 18 comprend au moins une paroi 36, 37, 38.

Par exemple, le deuxième caisson 18 comprend une pluralité de parois 36, 37, 38 définissant entre elles le deuxième logement 32.

Dans le mode de réalisation de l'invention illustré sur les Figures 1 et 2, la pluralité de parois du deuxième caisson 18 comprend par exemple deux parois longitudinales 36, deux parois transversales 37 reliant chacune les deux premières parois longitudinales 36, et un plafond 38.

Lorsque l'enceinte 10 est en position fermée (Figure 2), les parois longitudinales 36 du deuxième caisson 18 s'étendent par exemple perpendiculairement à l'axe transversal Y, les deux parois transversales 37 s'étendant perpendiculairement à l'axe longitudinal X et le plafond 38 s'étendant perpendiculairement à l'axe vertical Z.

Chacune des parois 36, 37, 38 du deuxième caisson 18 est par exemple une plaque, notamment sensiblement rectangulaire.

Dans une variante non-illustrée, le deuxième caisson 18 comprend une unique paroi délimitant le deuxième logement 32, qui est par exemple demi-cylindrique ou demi-sphérique.

La deuxième partition 34 ferme le deuxième logement 32.

Autrement dit, le deuxième logement 32 est un volume fermé.

Plus précisément, le deuxième logement 32 est délimité entre la ou les parois 36, 37, 38 du deuxième caisson 18 et la deuxième partition 34.

Comme cela est visible sur la Figure 2, le deuxième logement 32 est de préférence le symétrique du premier logement 20 selon le plan XY, lorsque l'enceinte 10 est en position fermée.

Dans le mode de réalisation particulier illustré, la deuxième partition 34 s'étend notamment en regard du plafond 38 du deuxième caisson 18.

De préférence, la deuxième partition 34 est fixée à chacune des deux parois longitudinales 36 et des deux parois transversales 37 du deuxième caisson 18, et notamment sur le bord inférieur de chacune de ces quatre parois 36,37 comme cela est visible sur les Figures.

Par bord inférieur, on entend ici le bord de chacune de ces quatre parois 36,37 opposé au plafond 38.

Comme cela est visible sur les Figures, la deuxième partition 34 comprend une face interne 34A, en particulier orientée vers le deuxième logement 32, et une face externe 34B. Les deux faces 34A, 34B sont par exemple opposées selon l'axe vertical Z, lorsque l'enceinte 10 est en position fermée.

La deuxième partition 34 est de préférence une plaque.

En particulier, l'épaisseur de la deuxième partition 34, c'est-à-dire la distance entre les deux faces 34A, 34B, est constante, et par exemple comprise entre 3 mm et 10 mm.

Comme cela est visible sur les Figures, le deuxième partition 34 est de préférence le symétrique de la première partition 22 selon le plan XY, lorsque l'enceinte 10 est en position fermée.

Avantageusement, la deuxième partition 34 comprend une partie centrale 40 apte à définir un logement de réception 41 de l'instrument médical 12.

Par exemple, la partie centrale 40 a une section transversale en forme de demi-cercle, par exemple de diamètre compris entre 10 cm et 30 cm.

Par exemple, le logement de réception 41 est délimité par la face externe 34B de la partie centrale 40 de la deuxième partition 34.

En particulier, le logement de réception 41 est par exemple en forme de demi-cylindre s'étendant selon l'axe longitudinal X, dans la position fermée.

Alternativement, dans des variantes-illustrées, la partie centrale 40 de la deuxième partition 34 a une section transversale en forme de U ou de forme rectangulaire.

Dans un mode de réalisation particulier, la deuxième partition 34 comprend également deux parties latérales 42 reliées entre elle par la partie centrale 40.

En particulier, chacune des parties latérales 42 de la deuxième partition 34 s'étend entre le bord inférieur de l'une des parois longitudinales 36 du deuxième caisson 18 et la partie centrale 40 de la deuxième partition 34.

De préférence, chacune des parties latérales 42 de la deuxième partition 34 s'étend parallèlement au plafond 38 du deuxième caisson 18.

La deuxième partition 34 est de préférence transparente aux rayonnements UV, et notamment aux rayonnements UV-C.

En particulier, la deuxième partition 34 a une transmittance aux rayonnements UV supérieure à 30%, et de préférence supérieure à 50%, et de manière encore plus préférentielle supérieure à 90%.

La deuxième partition 34 est préférablement fabriquée en quartz, de préférence du quartz synthétique, tel que par exemple un verre de quartz optique de catégorie JGS1.

Alternativement, la deuxième partition 34 est fabriquée en un polymère, préférablement un copolymère d'oléfine cyclique (COC de l'anglais « Cyclic olefin copolymer »), en particulier du TOPAS^{®} 8007X10, ou un polyméthylpentène, tel que le TPX^{™} RT18, ou de l'éthylène-propylène fluoré.

Comme cela est visible sur la Figure 1, dans la position ouverte, la première partition 22 et la deuxième partition 34 sont par exemple écartées l'une de l'autre.

Plus précisément, dans la position ouverte, les parties centrales 28, 40 des deux partitions 22, 34 sont écartées l'une de l'autre, en particulier de sorte que les deux logements de réception 29, 41 ne se touchent pas.

Comme cela est visible sur la Figure 2, dans la position fermée, la première 22 et la deuxième partition 34 définissent entre elles un volume de désinfection 44 fermé.

Plus précisément, dans la position fermée, les deux partitions 22, 34 s'étendent en regard l'une de l'autre.

Par exemple les deux parties centrales 28,40 s'étendent en regard l'une de l'autre, de sorte à définir entre elles le volume de désinfection 44 fermé, comme cela est visible sur la Figure 2.

Dans le mode de réalisation particulier illustré, les parties latérales 30, 42 des deux partitions 22, 34 s'étendent l'une contre l'autre dans la position fermée de l'enceinte 10.

En particulier, le volume de désinfection 44 se compose par exemple des deux logements de réception 29, 41.

Par exemple, comme cela est visible sur les Figures, le volume de désinfection 44 est cylindrique.

Alternativement, dans une variante non-illustrée dans laquelle une seule des deux partitions 22, 34 comprend une partie centrale définissant un logement de réception, le volume de désinfection est constitué uniquement de cet unique logement de réception.

De préférence, le volume de désinfection 44 est configuré pour s'adapter aux dimensions et aux formes variées de l'instrument médical 12.

De manière avantageuse, le volume de désinfection 44 est configuré de sorte à être assez grand pour recevoir l'instrument médical 12 en entier.

Le volume de désinfection 44 a par exemple un volume compris entre 3000 cm³ et 100 000 cm³.

L'enceinte 10 comprend en outre un dispositif 46 de génération de rayonnements UV configuré pour générer des rayonnements UV, et notamment des rayonnements UV-C, vers le volume de désinfection 44.

Par rayonnements UV-C, on entend des rayonnements caractérisés par une longueur d'onde comprise entre 200 et 280 nm
Le dispositif 46 de génération de rayonnements UV est arrangé dans au moins l'un du premier logement 20 et du deuxième logement 32.

Autrement dit, le dispositif 46 de génération de rayonnements UV n'est pas arrangé à l'intérieur du volume de désinfection 44.

Dans un mode de réalisation particulier, le dispositif 46 de génération de rayonnements UV comprend une pluralité de sources 48 UV arrangées dans au moins un du premier logement 20 et du deuxième logement 32.

Chaque source 48 est par exemple une lampe à LED (Light-Emitting Diode se traduisant en diode électroluminescente).

Avantageusement, des sources 48 UV sont arrangées à la fois dans le premier logement 20 et dans le deuxième logement 32.

De préférence, comme cela est visible sur la Figure 2, les sources 48 sont arrangées en regard de la partie centrale 28, 40 d'au moins une des partitions 22, 34.

De manière encore plus préférentielle, les sources 48 sont arrangées en regard de la partie centrale 28, 40 de chacune des deux des partitions 22, 34.

De manière avantageuse, les sources 48 sont reparties de manière uniforme tout autour du volume de désinfection 44, notamment de sorte à optimiser la répartition des rayonnements UV et permettre ainsi une désinfection uniforme de l'instrument médical 12 arrangé dans le volume de désinfection 44.

De manière optionnelle, le premier caisson 16 comprend par exemple également au moins un composant électronique additionnel 50, 52 agencé dans le premier logement 20.

En particulier, l'au moins un composant électronique additionnel est par exemple une unité d'identification 50 ou une unité de contrôle 52 de la désinfection.

L'unité d'identification 50 est par exemple configurée pour identifier l'instrument médical 12 à désinfecter, notamment pour assurer une traçabilité des désinfections.

Par « identifier », on entend plus particulièrement la lecture d'informations d'identification de l'instrument médical 12, telles que le numéro d'identification de l'instrument médical 12 et/ou le modèle de l'instrument médical 12.

Par exemple, l'unité d'identification 50 comprend un lecteur de radio-identification configuré pour lire les informations d'identification d'une étiquette d'identification fixée à l'instrument médical 12. L'étiquette d'identification comprend par exemple une puce de radio-identification, également nommée une puce RFID (de l'anglais « radio frequency identification »), contenant ces informations d'identification. La puce RFID est par exemple une puce à basse fréquence, une puce à haute-fréquence, par exemple du type puce NFC (Near-Field Communication, se traduisant en communication en champ proche), ou une puce UHF (Ultra Haute Fréquence).

L'unité de contrôle 52 de la désinfection est par exemple un calculateur propre à contrôler le procédé de désinfection par l'enceinte de désinfection.

Par exemple, l'unité de contrôle 52 est un circuit électronique conçu pour manipuler et/ou transformer des données représentées par des quantités électroniques ou physiques dans des registres du calculateur et/ou des mémoires en d'autres données similaires correspondant à des données physiques dans les mémoires de registres ou d'autres types de dispositifs d'affichage, de dispositifs de transmission ou de dispositifs de mémorisation.

En tant qu'exemples spécifiques, l'unité de contrôle 52 est réalisée sous forme d'un composant logique programmable, tel qu'un FPGA (de l'anglais *Field Programmable Gate Array*), ou encore d'un circuit intégré, tel qu'un ASIC (de l'anglais *Application Specific Integrated Circuit*).

En variante, lorsque le procédé est réalisé sous forme d'un ou plusieurs logiciels, c'est-à-dire sous forme d'un programme d'ordinateur, également appelé produit programme d'ordinateur, il est en outre apte à être enregistré sur un support, non représenté, lisible par ordinateur. Le support lisible par ordinateur est par exemple un medium apte à mémoriser des instructions électroniques et à être couplé à un bus d'un système informatique. A titre d'exemple, le support lisible est un disque optique, un disque magnéto-optique, une mémoire ROM, une mémoire RAM, tout type de mémoire non-volatile (par exemple FLASH ou NVRAM) ou une carte magnétique. Sur le support lisible est alors mémorisé un programme d'ordinateur comprenant des instructions logicielles.

Plus précisément, l'unité de contrôle 52 est configurée pour contrôler le dispositif 46 de génération de rayonnements UV, et par exemple pour contrôler chacune des sources 48.

Par exemple, l'unité de contrôle 52 est configurée pour mettre en œuvre différents cycles de désinfection différents, correspondant par exemple à des doses et/ou des durées différentes.

En particulier, l'unité de contrôle 52 de la désinfection est configurée pour mettre en œuvre au moins un cycle de désinfection haut-niveau et un cycle de stérilisation.

Par désinfection haut-niveau, communément appelée High-Level Désinfection (HLD), on entend ici une désinfection impliquant que l'ensemble de micro-organismes viables doivent être tués, à l'exception d'un petit nombre de spores.

De manière optionnelle, l'enceinte 10 comprend en outre un panneau de commande 54, en particulier connecté à l'unité de contrôle 52, et de préférence arrangée sur le premier caisson 16.

Le panneau de commande 54 comprend par exemple un écran et/ou des moyens de contrôle, tel que des boutons par exemple.

Le panneau de commande 54 permet par exemple de choisir le cycle de désinfection et/ou d'assurer un suivi de la désinfection.

En outre, l'enceinte 10 comprend par exemple également un moyen d'alimentation électrique 56.

Le moyen d'alimentation électrique 56 comprend par exemple une batterie ou un câble d'alimentation.

Un procédé de désinfection de l'instrument médical 12 dans l'enceinte de désinfection 10 va maintenant être décrit.

Premièrement, une enceinte de désinfection 10 telle que décrite ci-dessus est fournie.

Deuxièmement, l'enceinte de désinfection 10 est passée en position ouverte.

Ensuite, l'instrument médical 12 destiné à être désinfecté est positionné dans l'enceinte de désinfection 10.

Plus précisément, l'instrument médical 12 est posé sur l'une des partitions 22, 34, et en particulier sur la face externe 22B, 34B de l'une des partitions 22, 34, et notamment sur l'une des parties centrales 28, 40, et plus précisément dans l'un des logements de réception 29, 41.

Par exemple, l'instrument médical 12 est posé sur la face externe 22B de la partie centrale 28 de la première partition 22, comme cela est visible sur la Figure 2.

De préférence, l'instrument médical 12 est identifiée par l'unité d'identification 50.

L'enceinte de désinfection 10 est ensuite passée en position fermée.

Plus précisément, le deuxième caisson 18 est déplacé vers sa position fermée, de sorte notamment à fermer le volume de désinfection 44.

Un cycle de désinfection par rayonnements UV générée par le dispositif 46 de génération de rayonnements UV est ensuite mis en œuvre, notamment par l'unité de contrôle 52.

Une telle enceinte de désinfection 10 est particulièrement avantageuse pour la désinfection d'instruments médicaux 12, et en particulier de sondes médicales.

En effet, grâce aux partitions 22, 34 servant de support à l'instrument médical 12, une telle enceinte 10 permet de désinfecter une large gamme d'instruments, de tailles et de formes variées, qu'ils soient équipés ou non de câbles.

En outre, les partitions 22, 34 évitent que l'instrument médical touche les parois de l'enceinte 10, ce qui permet de limiter la création de zones non désinfectées (également appelées cold spot).

Par ailleurs, les partitions 22, 34 jouent un rôle de barrière entre les sources UV et l'instrument médical, ce qui permet d'éviter de les endommager.

L'homme du métier comprendra que les modes de réalisation et variantes précédemment décrits peuvent être combinés entre eux pourvu qu'ils soient compatibles techniquement.

## Revendications

1. Enceinte (10) de désinfection d'un instrument médical (12), l'enceinte (10) comprenant :
- un premier caisson (16) définissant un premier logement (20) fermé par une première partition (22), et
- un deuxième caisson (18) définissant un deuxième logement (32) fermé par une deuxième partition (34);
le deuxième caisson (18) étant mobile par rapport au premier caisson (16) entre une position ouverte et une position fermée,
dans la position fermée, la première partition (22) et la deuxième partition (34) définissant entre elles un volume de désinfection (44) fermé ;
l'enceinte (10) comprenant en outre un dispositif (46) de génération de rayonnements UV configuré pour générer des rayonnements UV dans le volume de désinfection (44), le dispositif (46) de génération de rayonnements UV étant arrangé dans au moins l'un du premier logement (20) et du deuxième logement (32).

2. Enceinte (10) selon la revendication 1, dans laquelle au moins l'une de la première partition (22) et de la deuxième partition (34) comprend une partie centrale (28, 40) apte à définir un logement de réception (29, 41) de l'instrument médical (12).

3. Enceinte (10) selon la revendication 1 ou 2, dans laquelle au moins l'une de la première (22) et de la deuxième partition (34) est transparente aux rayonnements UV.

4. Enceinte (10) selon la revendication 3, dans laquelle au moins l'une de la première (22) et de la deuxième (34) partition a une transmittance aux rayonnements UV supérieure à 30%, et de préférence supérieure à 50%.

5. Enceinte (10) selon l'une quelconque des revendications précédentes, dans laquelle au moins l'une de la première (22) et de la deuxième partition (34) est fabriquée en quartz.

6. Enceinte (10) selon l'une quelconque des revendications précédentes, comprenant en outre une charnière (19) agencée entre le premier caisson (16) et le deuxième caisson (18) et autorisant la mobilité du deuxième caisson (18) par rapport au premier caisson (16).

7. Enceinte (10) selon l'une quelconque des revendications précédentes, dans laquelle le volume de désinfection (44) est cylindrique.

8. Enceinte (10) selon l'une quelconque des revendications précédentes, dans laquelle le dispositif (46) de génération de rayonnements UV comprend une pluralité de sources UV (48) arrangées dans au moins un du premier logement (20) et du deuxième logement (32).

9. Enceinte (10) selon l'une quelconque des revendications précédentes, dans laquelle le premier caisson (16) comprend au moins un composant électronique additionnel (50, 52) agencé dans le premier logement (20).

10. Enceinte (10) selon la revendication 9, dans laquelle, dans laquelle l'au moins un composant électronique est une unité d'identification (50) ou une unité de contrôle (52) de la désinfection.
